# EUROPEAN PATENT APPLICATION

(11) **EP 2 377 495 A1**
(43) Date of publication of application: **19.10.2011**
(21) Application number: 10305406.0
(22) Date of filing: 19.04.2010
(51) Int. Cl.: A61F 2/44

(54) **Spinal implant**

(71) Applicant: Warsaw Orthopedic, Inc., Warsaw, IN 46581 (US)
(72) Inventor: Liu, Mingyan, 92340 Bourg-La-Reine (FR); Josse, Loic, Yens VD1169 (CH); Maxy, Phillippe, Les Marais 39400 Morbier (FR)
(74) Representative: Kühn, Armin

(57) **Abstract**

A spinal implant is disclosed. The spinal implant comprises a first component, a second component, a core and a tether. The first component comprises a first exterior surface configured for engaging a first vertebra, and a first interior surface opposing the first exterior surface. The second component comprises a second exterior surface configured for engaging a second vertebra, and a second interior surface opposing the second exterior surface. The core is configured to interface with the first interior surface and the second interior surface, and the tether configured to contact the first component and the second component. The tether is material for maintaining the core in place between the first and second components, and the tether is not configured to attach to the first component or the second component.

## Description

### FIELD OF INVENTION

The present invention is directed to spinal implants.

### BACKGROUND

The present disclosure relates to spinal implants, and particularly, spinal implants for intervertebral space.

### SUMMARY OF THE INVENTION

A spinal implant is disclosed. The spinal implant comprises a first component, a second component, a core and a tether. The first component comprises a first exterior surface configured for engaging a first vertebra, and a first interior surface opposing the first exterior surface. The second component comprises a second exterior surface configured for engaging a second vertebra, and a second interior surface opposing the second exterior surface. The core is configured to interface with the first interior surface and the second interior surface, and the tether configured to contact the first component and the second component. The tether is material for maintaining the core in place between the first and second components, and the tether is not configured to attach to the first component or the second component.

Additional aspects and features of the present disclosure will be apparent from the detailed description and claims as set forth below.

### BRIEF DESCRIPTION OF THE DRAWINGS

Figure 1 is an isometric view of a spinal implant for placement in a disc space between adjacent vertebral bodies;

Figure 2 is an exploded, isometric view of the spinal implant of Figure 1;

Figure 3 is a cross-sectional, isometric view of implant along and in the direction of line III-III of Figure 1;

Figure 4 is an isometric view of the core of the implant of Figure 1;

Figure 5 is an isometric view of the second component of the implant of Figure 1;

Figure 6 is a front view of the implant of Figure 1 after it is implanted in a disc space between adjacent vertebral bodies;

Figure 7 is a cross-sectional, isometric view of an implant;

Figure 7A is an isometric view of a core of the implant of Figure 7;

Figure 7B is an isometric view of a second component of the implant of Figure 7;

Figure 8 is a cross-sectional, isometric view of an implant;

Figure 8A is an isometric view of a core of the implant of Figure 8;

Figure 8B is an isometric view of a second component of the implant of Figure 8;

Figure 9 is a cross-sectional, isometric view of an implant;

Figure 9A is an isometric view of a core of the implant of Figure 9;

Figure 9B is an isometric view of a second component of the implant of Figure 9;

Figure 10 is a cross-sectional, isometric view of an implant;

Figure 10A is an isometric view of a core of the implant of Figure 10;

Figure 10B is an isometric view of a second component of the implant of Figure 10;

Figure 11 is a cross-sectional, isometric view of an implant;

Figure 11A is an isometric view of a core of the implant of Figure 11;

Figure 11B is an isometric view of a second component of the implant of Figure 11;

Figure 12 is a cross-sectional, isometric view of an implant;

Figure 12A is an isometric view of a core of the implant of Figure 12; and

Figure 12B is an isometric view of a second component of the implant of Figure 12.

### DETAILED DESCRIPTION

For the purposes of promoting an understanding of the principles of the invention, reference will now be made to the embodiments, or examples, illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

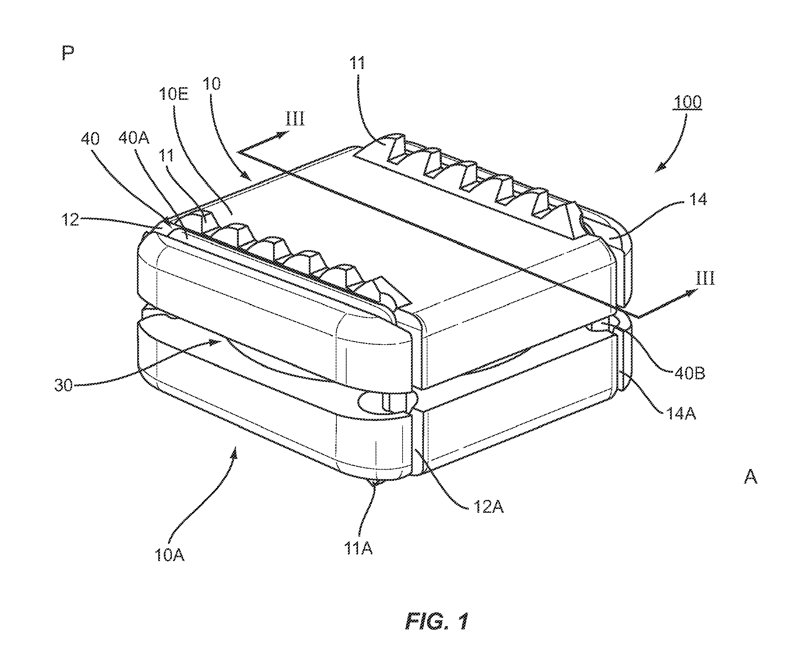

Figure 1 shows an isometric view of a spinal implant 100 for placement in a disc space between adjacent vertebral bodies. The implant 100 comprises a first component 10, a second component 10A, a core 30 and a tether 40. As shown in Figure 1, the tether 40 comprises a first tether component 40A and a second tether component 40B. The first component 10 comprises a first exterior surface 10E configured for engaging a first vertebra, and a first interior surface opposing the first exterior surface 10E. The second component 10A comprises a second exterior surface configured for engaging a second vertebra, and a second interior surface opposing the second exterior surface. The core 30 is configured to interface with the first interior surface and the second interior surface. The tether 40 is configured to contact the first component 10 and the second component 10A, wherein the tether 40 is material for maintaining the core 30 in place between the first and second components 10 and 10A, and wherein the tether 40 is not configured to attach to the first component 10 or the second component 10A.

As shown in implant 100 of Figure 1, the core 30 is not attached to the first interior surface or the second interior surface. Thus, as shown, the core 30 is not attached to the first component 10 or the second component 10A. Also, as shown in Figure 1, the tether 40 is not attached to the first component 10 or the second component 10A. Further, as shown in Figure 1, the tether 40 is not attached to the core 30.

As shown in Figure 1, the tether 40 is configured to contact the first exterior surface 10E and the second exterior surface. More specifically, as shown in Figure 1, the first component 10 has a first recess 12 and a second recess 14 for maintaining the tether 40 in position for maintaining the core 30 in place between the first interior surface and the second interior surface. Similarly, as shown in Figure 1, the second component 10A has a first recess 12A and a second recess 14A for maintaining the tether 40 in position for maintaining the core 30 in place between the first interior surface and the second interior surface.

As shown in Figure 1, implant 100 further comprises structures 11 to help affix the first component 10 to a vertebral body. Structure 11 may be anchors, keels, spikes, pegs, teeth, prongs, ridges, or similar structures to help component 10 affix to a vertebral body. As shown in Figure 1, structures 11 are shown as a plurality or ridge of teeth. Similarly, as shown, structures 11A help affix the second component 10A to a vertebral body. As depicted in Figure 1, reference marker A represents an anterior side of a vertebral body, whereas reference marker P represents a posterior side of a vertebral body after implant 100 is implanted in its intended position. Specifically, as shown, implant 100 may be inserted from the anterior side of the vertebral bodies with a posterior side of the implant 100 (the side closer to reference marker P) entering the disc space prior to an anterior side of the implant 100 (the side closer to reference marker A). Surface coatings such as Hydroxyapatite (HA), plasma spray or beading also may be used instead of or in addition to respective structure 11 or 11A to help affix their respective components 10 or 10A to their respective vertebral bodies.

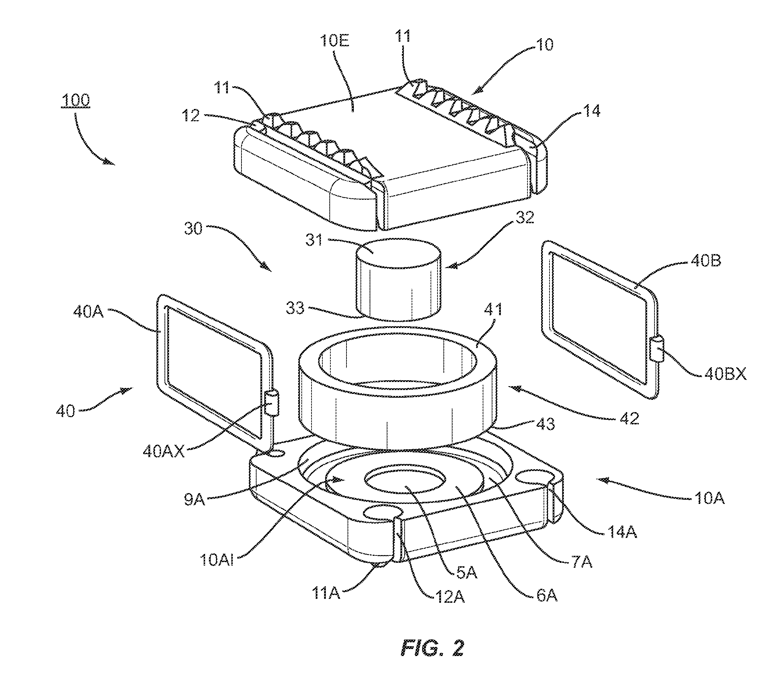

Figure 2 shows an exploded, isometric view of the spinal implant 100 of Figure 1. The implant 100 comprises a first component 10, a second component 10A, a core 30 and a tether 40. As shown in Figure 2, the core 30 comprises a first core component 32 and a second core component 42. Also, as schematically shown in Figure 2, the first core component 32 and the second core component 42 are distinct portions of the core 30 and are not attached to each other. Further, as shown in Figure 2, the first core component 32 is located in the substantial center of the implant 100.

Also, as shown in Figure 2, the first tether component 40A and the second tether component 40B are distinct portions of the tether 40 and are not attached to each other. The tether components 40A and 40B not being attached to each other and not attached to the first and second components 10 and 10A may help in manufacturing of the implant 100, may help provide improved motion of the implant 100 and may help better control rotational movements of the implant 100. Also, as shown in Figure 2, the first tether component 40A and the second tether component 40B have areas 40AX and 40BX, respectively, in order to form respective loops of tether material. Areas 40AX and 40BX may represent a knot, fusing together or other manner of joining the tether material, but note that the first tether component 40A and the second tether component 40B may not need distinct areas such as areas 40AX and 40BX that are schematically shown larger than the other portions of the tethers 40A and 40B, respectively, to form respective loops of tether material.

The term "substantial" (or "substantially") as used herein may be applied to modify any quantitative representation which could permissibly vary without resulting in a change in the basic function to which it is related. For example, while the first core component 32 is not necessarily located in the exact center of the implant 100, it is located in the substantial center of the implant 100. Specifically, the exact center in all planes of the implant 100 may be difficult to determine, but at least in the embodiment of Figure 2 as shown, the first core component is located in the substantial center of the implant 100, and in the case of spinal implants, this location of the first core component 32 helps the implant 100 properly perform one of its functions of maintaining disc height (i.e., the proper distance between the adjacent vertebrae).

The spinal implant 100 may serve as a fusion device or may serve as a motion-preserving device. In the case of a motion-preserving implant, the first core component 32 and the second core component 42 provide a degree of flexibility or compliance to the spinal implant 100. The first core component 32 and the second core component 42 may be made of the same material or may be made of different materials. Suitable materials include, but are not limited to, any one or any combination of a metal, polymer, ceramic, or other biocompatible material. In the case of a motion-preserving implant, a suitable material for the first core component 32 and/or the second core component 42 may be a viscoelastic material, and more specifically, may be any one or combination of polycarbonurethane ("PCU"), polyurethane ("PU"), polyethelene, silicone and hydrogel. Further, the first core component 32 and the second core component 42 may have different material properties. For example, if made of the same or different materials, the first core component 32 may be harder than the second core component 42. When, for example, the first core component 32 is harder than the second component 42, the first core component 32 may help maintain disc height and the second core component 42 may help balance the implant 100 and help the implant's overall motion, e.g., help it achieve desired flexion, extension and rotation.

The first component 10 and the second component 10A may be made of any metal or non-metal biocompatible material. Suitable materials include, but are not limited to, any one or combination of Titanium Alloys, commercially available Titanium, stainless steel, polyetheretherketone ("PEEK"), cobalt chrome ("CoCr"), polyethylene, and ultra high molecular weight polyethylene ("UHMWPE").

The tether 40 may be made of any one or combination of a cloth, metal, solid polymer, fabric, mesh, or other biocompatible material. Some polymer materials may include any one or combination of polyethylene, polyester, polyvinyl, polyvinyl alcohol, polyacrylonitrile, polyamide, polytetrafluoroethylene, poly-paraphenylene and terephthalamide. Further, the tether 40 may be made of a suture wire of polyester or polyethylene. In addition, the material for the tether 40 may be elastic, woven, knitted, braided or flexible. Some woven, knitted or braided materials may, for example, include nylon, Dacron®, and/or woven fibers or filaments of polyester, polyethelene, polypropylene, polyetheretherketone ("PEEK"), polytetrafluoroethylene ("PTFE"), woven PEEK, and/or Bionate® or Pursil® manufactured by DMS PTG, Inc. of Berkeley, California. Some elastic materials may, for example, include latex, rubber, silicone, polyurethane, silicone-polyurethane copolymers, and/or polyolefin rubbers. Other suitable materials may, for example, include Gore-Tex®, Kevlar®, Spectra, polyether, polycarbonate urethane, shape memory material with pseudo elastic or superelastic characteristics, metals, metal alloys, and polymers, braided polymers, synthetic resorbable materials such as polyactide, polygycolide, polyorthoester, calcium phosphate, and/or glass, nonresorbable polyethylene, cellulose, materials that are potentially absorbable, and/or materials that are used in making artificial ligaments. Further, suitable materials should be non-biodegradable and non-resorbable. In addition to woven, braided, or knitted structures, the tether 40 also may be composed of non-woven structures such as non-woven mesh, or chained structures.

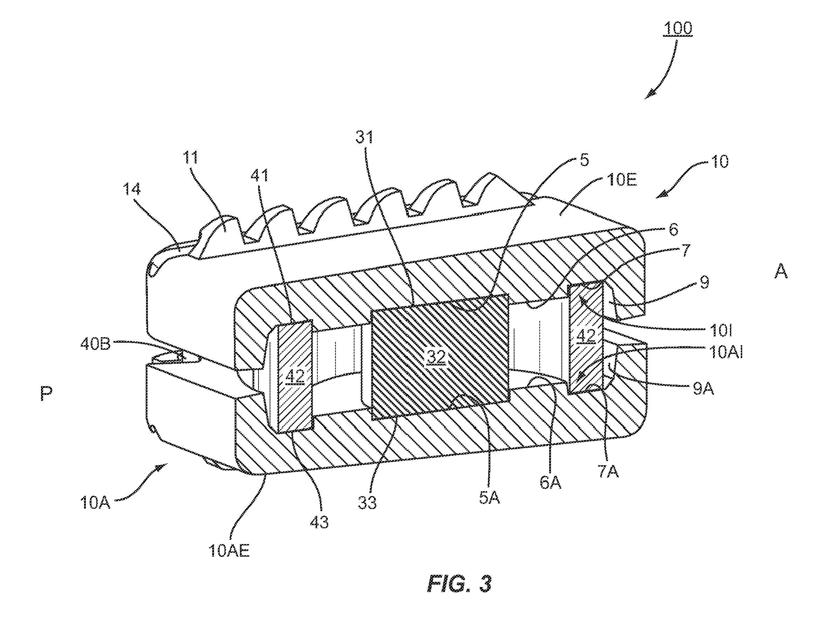

Figure 3 shows a cross-sectional, isometric view of implant 100 along and in the direction of line III-III of Figure 1. Figure 3 shows the first exterior surface 10E and the first interior surface 10I of the first component 10. As shown in Figure 3, the first exterior surface 10E comprises the structures 11 and the second recess 14. Also visible in Figure 3 is second tether component 40B. As shown in Figure 3, the first interior surface 10I comprises a first central inner surface 5, a first annular inner surface 7, and a first separation surface 6 located between the central inner surface 5 and the annular inner surface 7. Also, as shown in Figure 3, the first interior surface 10I further comprises a first wall surface 9. As shown in Figure 3, first wall surface 9 comprises two constituent wall surfaces. First wall surface 9, however, may take on other shapes or forms and may, for example, comprise a single wall surface situated at a right angle to annular surface 7. Similarly, first interior surface 10I and its constituent surfaces may take on different shapes and forms than that shown in Figure 3. As shown in Figure 3, interior surface 10I helps maintain the core 30 in place between the first and second components 10 and 10A and helps maintain the core components 32 and 42 in proper position.

Also, Figure 3 shows the second exterior surface 10AE and the second interior surface 10AI of the second component 10A. As shown in Figure 3, the second interior surface 10AI comprises a second central inner surface 5A, a second annular inner surface 7A, and a second separation surface 6A located between the second central inner surface 5A and the second annular inner surface 7A. Also, as shown in Figure 3, the second interior surface 10AI further comprises a second wall surface 9A. As shown in Figure 3, and as with first wall surface 9, second wall surface 9A comprises two constituent wall surfaces. Similarly, second wall surface 9A, however, may take on other shapes or forms and may, for example, comprise a single wall surface situated at a right angle to annular surface 7A. Similarly, second interior surface 10AI and its constituent surfaces may take on different shapes and forms than that shown in Figure 3. As shown in Figure 3, interior surface 10AI helps maintain the core 30 in place between the first and second components 10 and 10A and helps maintain the core components 32 and 42 in proper position.

Also, Figure 3 shows that the first core component 32 and the second core component 42. As shown in Figure 3, the first core component 32 comprises a superior surface 31 and an inferior surface 33. Similarly, the second core component 42 comprises a superior surface 41 and an inferior surface 43. As shown in Figure 3, the superior surface 31 of the first core component 32 contacts the first central inner surface 5, and the inferior surface 33 of the first core component 32 contacts the second central inner surface 5A. Further, as shown in Figure 3, the superior surface 41 of the second core component 42 contacts the first annular inner surface 7, and the inferior surface 43 of the second core component 42 contacts the second annular inner surface 7A.

Implant 100 as shown in Figure 3 is designed for a disc space requiring a certain amount of lordosis. That is, the thickness of the first component 10 and the second component 10A increases from the posterior side to the anterior side. Specifically, with respect to the first component 10, for example, the distance between the first exterior surface 10E and the first interior surface 10I increases from the posterior side to the anterior side. Although implant 100 as shown is designed to accommodate a disc space having a certain amount of lordosis, implant 100 may be designed to accommodate a disc space having a certain amount of kyphosis (wherein the thickness of implant 100 or either one or both of components 10 and 10A decreases from the posterior side to the anterior side) or neither lordosis or kyphosis (wherein the thickness of implant 100 and the thickness of each of the components 10 and 10A does not vary from the posterior side to the anterior side).

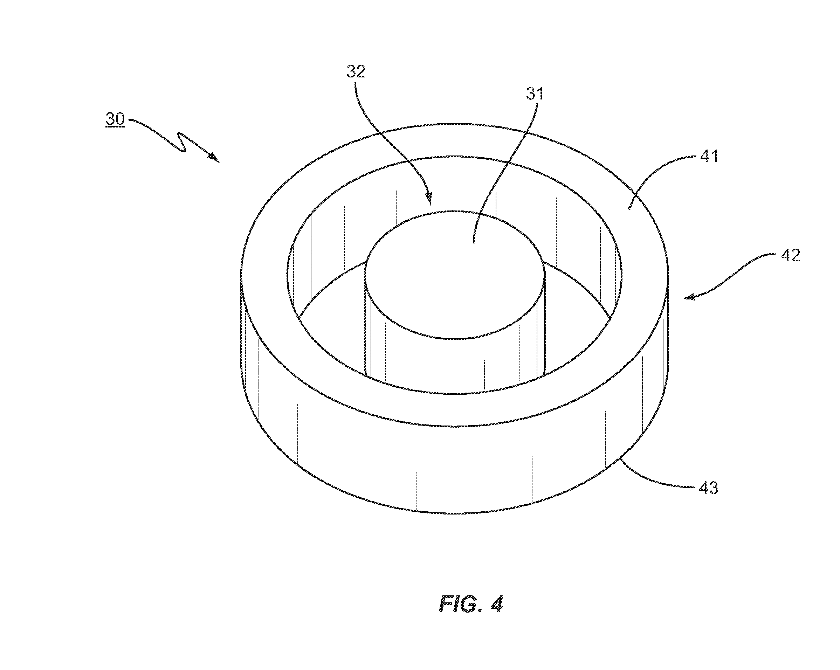

Figure 4 shows an isometric view of the core 30 of implant 100. Specifically, Figure 4 shows the first core component 32 and the second core component 42. Further, Figure 4 shows the superior surface 31 of the first core component 32 as well as the superior surface 41 and inferior surface 43 of the second core component 42.

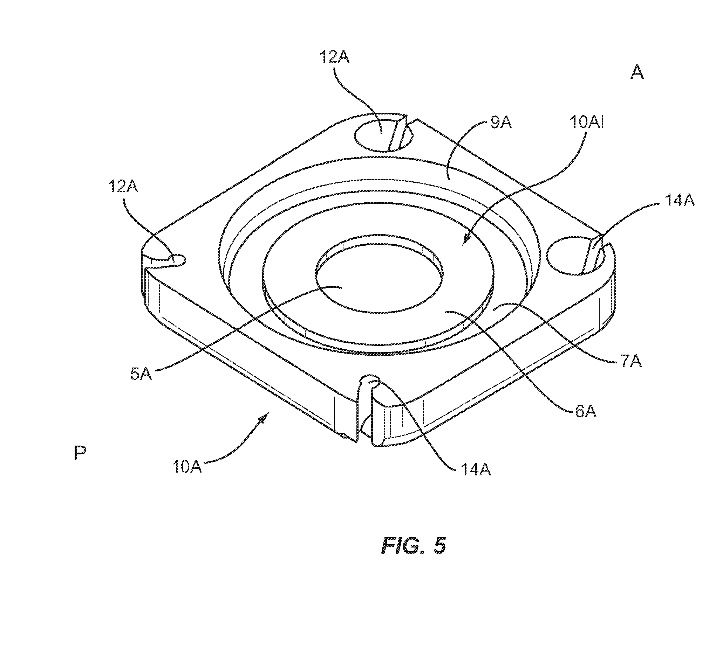

Figure 5 shows an isometric view of the second component 10A of implant 100. Specifically, Figure 5 shows the second interior surface 10AI of the second component 10A. Figure 5 further shows the second wall surface 9A, the second central inner surface 5A, the second annular inner surface 7A, and the second separation surface 6A located between the second central inner surface 5A and the second annular inner surface 7A. Also, Figure 5 shows respective parts of the first recess 12A and the second recess 14A for maintaining the tether 40 in position for maintaining the core 30 in place between the first interior surface 10I and the second interior surface 10AI. As shown in Figure 5, the parts of the recesses 12A and 14A that are located on the anterior side of the implant 100 are larger than those parts of the recesses 12A and 14A that are located on the posterior side of the implant 100. In the case of implant 100, the larger parts of recesses 12A and 14A are intended to accommodate areas 40AX and 40BX of tether 40, respectively. Note that it is possible to form the first tether component 40A and a second tether component 40B with a constant cross-sectional area or thickness throughout so that the parts of the recesses 12A and 14A on the anterior side of the implant 100 may be of the same size of the parts of the recess 12A and 14A on the posterior side of the implant 100.

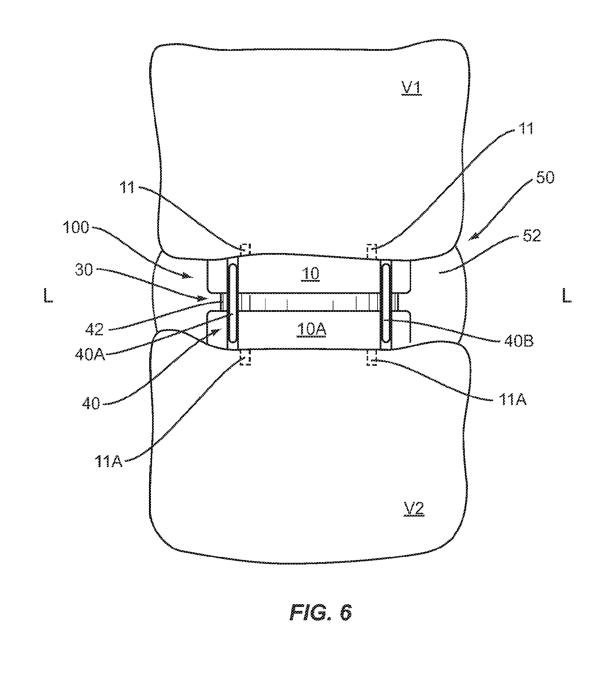

Figure 6 shows a front view of spinal implant 100 in its intended position after it is implanted in a disc space 50 between adjacent vertebral bodies V1 and V2. Specifically, Figure 6 shows implant 100 in position between vertebral bodies V1 and V2 from the anterior direction A. As depicted in Figure 6, reference markers L represent lateral sides of the vertebral bodies V1 and V2. Figure 6 shows the first component 10, the second component 10A, the core 30 and the tether 40. Specifically, Figure 6 shows the second core component 42 of core 30 and the first tether component 40A and the second tether component 40B of tether 40. The first exterior surface 10E engages vertebral body V1 and the second exterior surface 10AE engages second vertebral body V2. Also, Figure 6 shows structures 11 and 11A to help respective components 10 and 10A affix to vertebral bodies V1 and V2, respectively. Further, as shown in Figure 6, disc space 50 comprises tissue 52. Note that while implant 100 may be implanted in a disc space 50 and certain tissue 52 may be left intact to, for example, promote healing of the affected area, such tissue 52 may be completely removed so that only implant 100 resides in the disc space 50 after implantation. Tissue 52 may comprise, for example, annulus fibrosus tissue that surrounds nucleus pulposus tissue of an intervertebral disc.

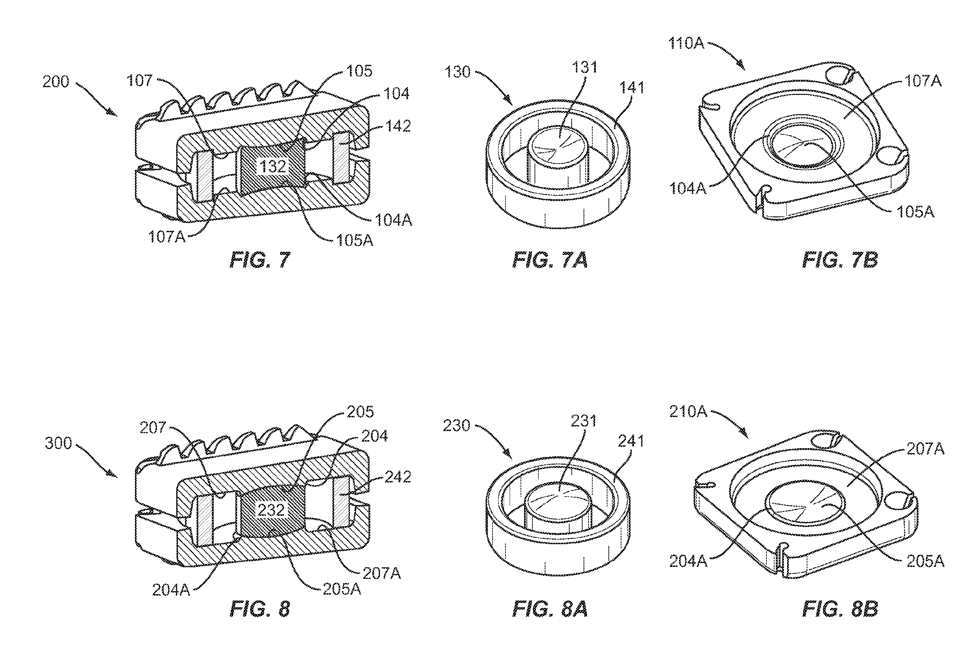

As mentioned with respect to Figures 3, 4 and 5, the first and second interior surface 101 and 10AI and its constituent surfaces may have different shapes and forms than that shown in those previous figures. For example, Figures 7-12 show various implants, each depicting a different embodiment of first and second interior surfaces and core combinations.

Figure 7 shows a cross-sectional, isometric view of implant 200 (along and in the direction of a line similar to that of III-III of Figure 1). Figure 7A shows an isometric view of a core 130 of implant 200. Figure 7B shows an isometric view of a second component 110A of implant 200. Specifically, Figures 7 and 7A show a core 130 that comprises a first core component 132 and a second core component 142. Further, Figures 7 and 7A show a superior surface 131 of the first core component 132 and the superior surface 131 has a substantially concave shape. Figures 7 and 7A show a superior surface 141 of the second core component 142 and the superior surface 141 has a substantially flat shape. Also, Figure 7 shows a first central inner surface 105, a first annular inner surface 107, and a first angled wall 104 adjacent the central inner surface 105. Similarly, Figures 7 and 7B show a second central inner surface 105A, a second annular inner surface 107A, and a second angled wall 104A adjacent the central inner surface 105A. As shown in Figures 7, 7A and 7B, central inner surfaces 105 and 105A are shaped (specifically having substantially convex surfaces) to accommodate the first core component 132, and annular surfaces 107 and 107A are shaped (specifically having substantially flat surfaces) to accommodate the second core component 142. The angled walls 104 and 104A may, for example, help contain the first core component 132 in its intended position, i.e., at the substantial center of implant 200.

Figure 8 shows a cross-sectional, isometric view of implant 300 (along and in the direction of a line similar to that of III-III of Figure 1). Figure 8A shows an isometric view of a core 230 of implant 300. Figure 8B shows an isometric view of a second component 210A of implant 300. Specifically, Figures 8 and 8A show a core 230 that comprises a first core component 232 and a second core component 242. Further, Figures 8 and 8A show a superior surface 231 of the first core component 232 and the superior surface 231 has a substantially convex shape. Figures 8 and 8A show a superior surface 241 of the second core component 242 and the superior surface 241 has a substantially flat shape. Also, Figure 8 shows a first central inner surface 205, a first annular inner surface 207, and a first angled wall 204 adjacent the central inner surface 205. Similarly, Figures 7 and 7B show a second central inner surface 205A, a second annular inner surface 207A, and a second angled wall 204A adjacent the second central inner surface 205A. As shown in Figures 8, 8A and 8B, central inner surfaces 205 and 205A are shaped (specifically having substantially concave surfaces) to accommodate the first core component 232, and annular surfaces 207 and 207A are shaped (specifically having substantially flat surfaces) to accommodate the second core component 242. The angled walls 204 and 204A may, for example, help contain first core component 232 in its intended position, i.e., at the substantial center of implant 300.

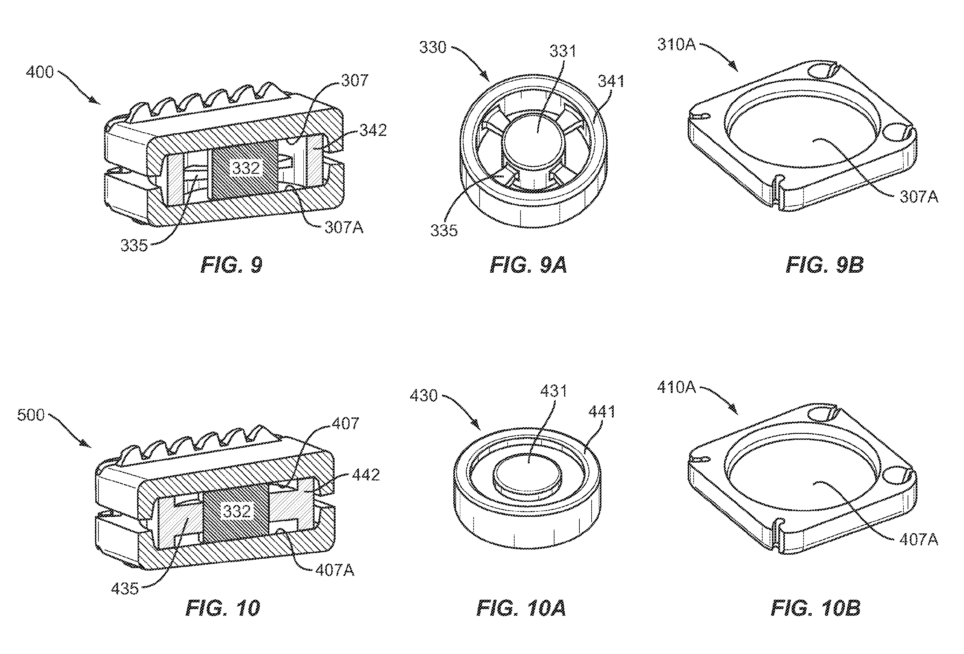

Figure 9 shows a cross-sectional, isometric view of implant 400 (along and in the direction of a line similar to that of III-III of Figure 1). Figure 9A shows an isometric view of a core 330 of implant 400. Figure 9B shows an isometric view of a second component 310A of implant 400. Specifically, Figures 9 and 9A show a core 330 that comprises a first core component 332, a second core component 342 and core members 335 connecting the first core component 332 to the second core component 342. Further, Figures 9 and 9A show a superior surface 331 of the first core component 332 and the superior surface 331 has a substantially flat shape. Figures 9 and 9A show a superior surface 341 of the second core component 342 and the superior surface 341 has a substantially flat shape. Also, Figure 9 shows a first inner surface 307, and Figures 9 and 9B show a second inner surface 307A. As shown in Figures 9, 9A and 9B, inner surfaces 307 and 307A are shaped (specifically having substantially flat surfaces) to accommodate the first core component 332 and the second core component 342.

Figure 10 shows a cross-sectional, isometric view of implant 500 (along and in the direction of a line similar to that of III-III of Figure 1). Figure 10A shows an isometric view of a core 430 of implant 500. Figure 10B shows an isometric view of a second component 410A of implant 500. Specifically, Figures 10 and 10A show a core 430 that comprises a first core component 432, a second core component 442 and a core member 435 connecting the first core component 432 to the second core component 442. Further, Figures 10 and 10A show a superior surface 431 of the first core component 432 and the superior surface 431 has a substantially flat shape. Figures 10 and 10A shows a superior surface 441 of the second core component 442 and the superior surface 441 has a substantially flat shape. Also, Figure 10 shows a first inner surface 407, and Figures 10 and 10B show a second inner surface 407A. As shown in Figures 10, 10A and 10B, inner surfaces 407 and 407A are shaped (having substantially flat surfaces) to accommodate the first core component 432 and the second core component 442.

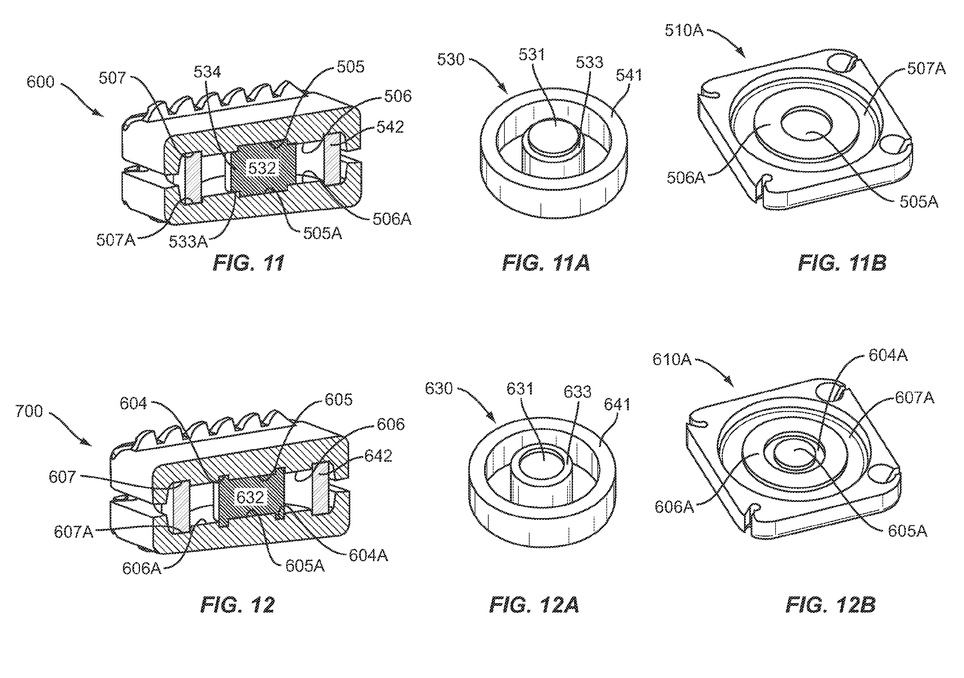

Figure 11 shows a cross-sectional, isometric view of implant 600 (along and in the direction of a line similar to that of III-III of Figure 1). Figure 11A shows an isometric view of a core 530 of implant 600. Figure 11B shows an isometric view of a second component 510A of implant 600. Specifically, Figures 11 and 11A show a core 530 that comprises a first core component 532 and a second core component 542. Further, Figures 11 and 11A show a first superior surface 531 and a second superior surface 533 of the first core component 532 and both the first superior surface 531 and the second superior surface 533 are substantially flat. Figures 11 and 11A show a superior surface 541 of the second core component 542 and the superior surface 541 has a substantially flat shape. Also, Figure 11 shows a first central inner surface 505, a first annular inner surface 507, and a first separation surface 506 located between the first central inner surface 505 and the first annular inner surface 507. Similarly, Figures 11 and 11B show a second central inner surface 505A, a second annular inner surface 507A, and a second separation surface 506A located between the second central inner surface 505A and the second annular inner surface 507A. As shown in Figures 11, 11A and 11B, central inner surfaces 505 and 505A and separation surfaces 506 and 506A are shaped (having substantially flat surfaces) to accommodate the first core component 532, and annular surfaces 507 and 507A are shaped (having substantially flat surfaces) to accommodate the second core component 542. The central inner surfaces 505 and 505A and separation surfaces 506 and 506A, and their respective orientation to each other may, for example, help contain the first core component 532 in its intended position, i.e., at the substantial center of implant 600.

Figure 12 shows a cross-sectional, isometric view of implant 600 (along and in the direction of a line similar to that of III-III of Figure 1). Figure 12A shows an isometric view of a core 630 of implant 700. Figure 12B shows an isometric view of a second component 610A of implant 700. Specifically, Figures 12 and 12A show a core 630 that comprises a first core component 632 and a second core component 642. Further, Figures 12 and 12A show a first superior surface 631 and a second superior surface 633 of the first core component 632 and both the first superior surface 631 and the second superior surface 633 have substantially flat shapes. Figures 12 and 12A show a superior surface 641 of the second core component 642 and the superior surface 641 has a substantially flat shape. Also, Figure 12 shows a first central inner surface 605, a first annular inner surface 607, and a first separation surface 606 located between the first central inner surface 605 and the first annular inner surface 607. Similarly, Figures 12 and 12B show a second central inner surface 605A, a second annular inner surface 607A, and a second separation surface 606A located between the second central inner surface 605A and the second annular inner surface 607A. As shown in Figures 12, 12A and 12B, central inner surfaces 605 and 605A and separation surfaces 606 and 606A are shaped (having substantially flat surfaces) to accommodate the first core component 632, and annular surfaces 607 and 607A are shaped (having substantially flat surfaces) to accommodate the second core component 642. The central inner surfaces 605 and 605A and separation surfaces 606 and 606A, and their respective orientation to each other may, for example, help contain first core component 632 in its intended position, i.e., at the substantial center of implant 700.

All adjustments and alternatives described above are intended to be included within the scope of the invention, as defined exclusively in the following claims. Those skilled in the art also should realize that such modifications and equivalent constructions or methods do not depart from the spirit and scope of the present disclosure, and that they may make various changes, substitutions, and alterations herein without departing from the spirit and scope of the present disclosure. For example, prostheses 100, 200, 300, 400, 500, 600 and 700 are shown as motion-preserving devices, but can be modified to be fusion devices. Similarly, although all of the prostheses above are described as being configured to affix to an endplate of a vertebral body and a vertical surface of a vertebral body that is an anterior surface of a vertebral body, all of the prostheses above may be configured to affix to a posterior surface of a vertebral body, a lateral surface of a vertebral body instead of or as well as an anterior surface of a vertebral body, or any plurality or combination or such surfaces.

Furthermore, as used herein, the terms components and modules may be interchanged. It is understood that all spatial references, such as "first," "second," "exterior," "interior," "superior," "inferior," "anterior," "posterior," "central," "annular," "outer," and "inner," and are for illustrative purposes only and can be varied within the scope of the disclosure.

## Claims

1. A spinal implant comprising:
a first component comprising:
a first exterior surface configured for engaging a first vertebra; and
a first interior surface opposing the first exterior surface;
a second component comprising:
a second exterior surface configured for engaging a second vertebra; and
a second interior surface opposing the second exterior surface;
a core configured to interface with the first interior surface and the second interior surface; and
a tether configured to contact the first component and the second component, wherein the tether is material for maintaining the core in place between the first and second components, and wherein the tether is not configured to attach to the first component or the second component.

2. The spinal implant of claim 1, wherein the core is not attached to the first interior surface or the second interior surface.

3. The spinal implant of claim 1 or 2, wherein the tether is not attached to the first component or the second component.

4. The spinal implant of one of claims 1 to 3, wherein the tether is not attached to the core.

5. The spinal implant of one of claims 1 to 4, wherein the core is not attached to the first component or the second component, and wherein the tether is not attached to the first component, the second component or the core.

6. The spinal implant of one of claims 1 to 5, wherein the core comprises a first core component and a second core component.

7. The spinal implant of claim 6, wherein the first core component and the second core component are distinct portions of the core and are not attached to each other.

8. The spinal implant of claim 6 or 7, wherein the first core component is located in the substantial center of the implant and wherein the first core component is preferably harder than the second core component.

9. The spinal implant of one of claims 1 to 8, wherein the spinal implant is a fusion device.

10. The spinal implant of one of claims 1 to 8, wherein the spinal implant is a motion-preserving device.

11. The spinal implant of one of claims 1 to 10, wherein the tether is configured to contact the first exterior surface and the second exterior surface.

12. The spinal implant of one of claims 1 to 11, wherein at least one of the first component and the second component further comprise a recess for maintaining the tether in position for maintaining the core in place between the first interior surface and the second interior surface.

## Amended claims

### Amended claims in accordance with Rule 137(2) EPC.

**1.** A motion-preserving spinal implant (100, 200, 300, 400, 500, 600, 700) for intervertebral space comprising:
a first component (10) comprising:
a first exterior surface (10E) configured for engaging a first vertebra; and
a first interior surface (10I) opposing the first exterior surface (10E);
a second component (10A; 110A; 210A; 310A; 410A; 510A; 610A; 710A) comprising:
a second exterior surface (10AE) configured for engaging a second vertebra; and
a second interior surface (10AI) opposing the second exterior surface (10AE);
a core (30; 130; 230; 330; 430; 530; 630) configured to interface with the first interior surface (10I) and the second interior surface (10AI), the core (30) being not attached to the first component (10) or the second component (10A), and the core (30) comprising a first core component (32; 132; 232; 332; 432; 532; 632) and a second core component (42; 142; 242; 342; 442; 542; 642) which are distinct portions and are not attached to each other, wherein the first core component (32; 132; 232; 332; 432; 532; 632) is located in the substantial center of the implant (100, 200, 300, 400, 500, 600, 700) and helps maintain disc space; and
a tether (40) configured to contact the first component (10) and the second component (10A), wherein the tether (40) is material for maintaining the core (30) in place between the first and second components (10, 10A), and wherein the tether (40) is not configured to attach to the first component (10) or the second component (10A), the tether (40) being not attached to the first component (10), the second component (10A) or the core (30), and the tether (40) comprising a first tether component (40A) and a second tether component (40B), the first and second tether components (40A, 40B) being distinct portions of the tether (40) and are not attached to each other, **characterized in that**
the first core component (32) is harder than the second core component (42), the second core component helping balance the implant (100).

**2.** The spinal implant (100, 200, 300, 400, 500, 600, 700) of claim 1, wherein the tether (40) is not attached to the first component (10) or the second component (10A).

**3.** The spinal implant (100, 200, 300, 400, 500, 600, 700) of claim 1 or 2, wherein the tether (40) is not attached to the core (30).

**4.** The spinal implant (100, 200, 300, 400, 500, 600, 700) of one of claims 1 to 3, wherein the tether (40) is configured to contact the first exterior surface (10E) and the second exterior surface (10AE).

**5.** The spinal implant (100, 200, 300, 400, 500, 600, 700) of one of claims 1 to 4, wherein at least one of the first component (10) and the second component (10A) further comprise a recess (12, 14) for maintaining the tether (40) in position for maintaining the core in place between the first interior surface (10I) and the second interior surface (10AI).
